# EUROPÄISCHE PATENTSCHRIFT

(11) **EP 1 120 396 B1**
(45) Veröffentlichungstag und Bekanntmachung des Hinweises auf die Patenterteilung: **15.10.2008**
(21) Anmeldenummer: 01101815.7
(22) Anmeldetag: 26.01.2001
(51) Int. Cl.: C07C 69/54, C07C 67/08, C07C 67/26, C08G 63/58, C09D 163/10, C08L 63/10

(54) **Verfahren zur Herstellung von strahlungshärtbaren Acrylaten**
Process for the preparation of radiation curable acrylates
Procédé de préparation d'acrylates photopolymérisables

(30) Priorität: 27.01.2000 DE 10003483
(43) Veröffentlichungstag der Anmeldung: 01.08.2001
(73) Patentinhaber: BASF SE, 67056 Ludwigshafen (DE)
(72) Erfinder: Königer, Rainer, Dr., 63329 Egelsbach (DE); Reich, Wolfgang, Dr., 67133 Maxdorf (DE); Paulus, Wolfgang, Dr., 55270 Ober-Olm (DE)
(74) Vertreter: Kinzebach, Werner

(56) Entgegenhaltungen:
- EP-A- 0 279 303
- DE-A- 3 316 593
- DE-A- 4 040 290
- US-A- 2 819 296
- US-A- 2 929 835
- US-A- 3 872 162

## Beschreibung

Die Erfindung betrifft ein Verfahren zur Herstellung von strahlungshärtbaren Acrylaten durch Umsetzung von Hydroxyverbindungen mit Acrylsäure oder Methacrylsäure und Umsetzung des Reaktionsproduktes mit einer Epoxidverbindung unter Verwendung von stark nucleophilen Aminen als Katalysator.

Aus der EP-A-54 105, der DE-A-33 16 593 und der EP-A-279 303 sind Verfahren bekannt, bei denen in einer ersten Stufe ein (Meth)acrylsäureester aus (Meth)acrylsäure und einer Hydroxyverbindung hergestellt und in einer zweiten Stufe unumgesetzte (Meth)acrylsäure mit Epoxiden umgesetzt wird. Als Katalysatoren für die Umsetzung in der zweiten Stufe sind in der DE-A-33 16 593 tertiäre Amine oder Lewis-Basen wie Thiodiglykol genannt. In der EP-A-54 105 wird Triphenylphosphin als Katalysator eingesetzt. In der EP-A-279 303 werden neben tertiären Aminen und Lewis-Basen in allgemeiner Form auch quartäre Ammoniumverbindungen genannt. Als tertiäre Amine sind Tributylamin und Dimethylethanolamin genannt.

In der US-A-2929835 werden Trialkylamine, wobei einer der Alkylreste für Cyclohexyl oder Aralkyl stehen kann, sowie heterocyclische tertiäre Amine als geeignete Katalysatoren für die Reaktion von Alkylenoxiden mit (Meth)acrylsäure genannt. Die DE 4040290 beschreibt ein zweistufiges Verfahren zur Herstellung von strahlungshärtbaren (Meth)acrylaten, durch Umsetzung von Hydroxyverbindungen mit (Meth)acrylsäure in Gegenwart eines sauren Katalysators und anschließenden Umsetzung des Reaktionsprodukts mit einer Epoxidverbindung in Gegenwart von N,N-Dimethylethanolamin.

Die bekannten Verfahren weisen einige Nachteile auf. Ammoniumverbindungen besitzen zwar hohe Aktivität, haben aber den Nachteil, daß sie im Vergleich zu den meisten Aminen relativ teuer sind und meist Halogene enthalten. Auf der anderen Seite sind die meisten Aminverbindungen relativ träge Katalysatoren für die Reaktion zwischen Carbonsäuren und Epoxiden. Hinzu kommt der unerwünschte Amingeruch im Produkt, eine stärkere Verfärbung des Produkts, und in einigen Fällen (bei Aminen mit niedrigem Molekulargewicht) kommt es zu Trübungen durch die Bildung von unlöslichen Salzen der Amine mit der Carbonsäure oder dem sauren Veresterungskatalysator.

Aufgabe der vorliegenden Erfindung ist es daher, ein Verfahren zur Herstellung von strahlungshärtbaren Acrylaten zur Verfügung zu stellen, das die gewünschten Acrylate in wirtschaftlicher Weise ergibt, d.h. es soll eine rasche Umsetzung unumgesetzter (Meth)acrylsäure mit einer Epoxidverbindung erfolgen. Außerdem sollen der Amingeruch und die Verfärbung des Produkts verringert werden.

Überraschenderweise wurde nun gefunden, daß diese Aufgabe durch Verwendung stark nucleophiler Amine als Katalysator für die Umsetzung mit dem Epoxid gelöst wird.

Die vorliegende Erfindung betrifft daher ein Verfahren zur Herstellung von strahlungshärtbaren Acrylaten durch Umsetzung einer Hydroxyverbindung mit Acrylsäure oder Methacrylsäure (1. Stufe), gegebenenfalls in Gegenwart eines sauren Katalysators, und Umsetzung des Reaktionsproduktes mit einer Epoxidverbindung in Gegenwart eines Katalysators (2. Stufe), das dadurch gekennzeichnet ist, daß man als Katalysator mindestens ein stark nucleophiles Amin der allgemeinen Formeln I oder II 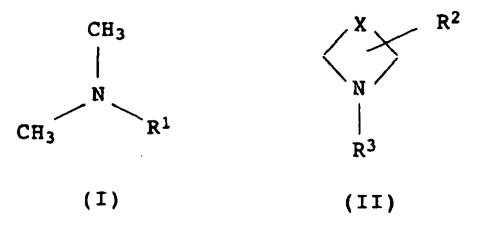 verwendet, wobei
R¹ für eine C₁-C₂₀-Alkylgruppe, bevorzugt C₄-C₁₈-Alkylgruppe, welche gegebenenfalls durch eine oder zwei Phenylgruppen substituiert sein kann, oder eine C₃-C₇-Cycloalkylgruppe steht,
R² für H oder einen C₁-C₁₂-Alkylrest steht,
X für CH₂, (CH₂)₂, (CH₂)₃, (CH₂)₄, CH₂OCH₂ oder CH₂NR⁴CH₂ steht,
R³ für C₁-C₄-Alkyl steht,
R⁴ für H oder C₁-C₄-Alkyl steht.

Bevorzugt sind die Dimethylalkylamine der Formel I.

Die Erfindung umfasst ferner die nach diesem Verfahren erhältlichen Acrylate sowie strahlungshärtbare Zubereitungen, welche diese Acrylate enthalten.

Beim erfindungsgemäßen Verfahren wird in einer 1. Stufe Acrylsäure oder Methacrylsäure (zusammenfassend (Meth)acrylsäure genannt) mit einer Hydroxyverbindung umgesetzt.

Als Hydroxyverbindungen in Betracht kommen Verbindungen mit einer oder mehreren Hydroxygruppen, insbesondere aliphatische Hydroxyverbindungen.

Als hydroxylgruppenhaltige Verbindungen mit einer Hydroxylgruppe pro Molekül eignen sich C₅-C₃₀-Monoalkohole, vorzugsweise C₈-C₂₀-Monoalkohole, beispielsweise 2-Ethylhexanol, Laurylalkohol, Stearylalkohol, 4-t-Butylcyclohexanol, 3,3,5-Trimethylcyclohexanol, 2-Methyl-3-phenylpropan-1-ol und Phenylglykol.

Geeignet sind auch entsprechende Oxalkylierungsprodukte, vorzugsweise Oxethylierungs- und Oxpropylierungsprodukte von C₂-C₁₂-Monoalkoholen. Der Oxalkylierungsgrad liegt in der Regel zwischen 1 bis 10, vorzugsweise zwischen 1 bis 5.

Die vorstehend genannten hydroxylgruppenhaltigen Verbindungen mit einer Hydroxylgruppe pro Molekül weisen vorzugsweise Molekulargewichte zwischen 130 und 300 auf.

Als hydroxylgruppenhaltige Verbindungen mit zwei oder mehreren Hydroxylgruppen pro Molekül eignen sich beispielsweise zwei- bis sechswertige C₂-C₂₀-Polyole, vorzugsweise C₂-C₁₀-Polyole, beispielsweise Diole, wie Ethylenglykol, 1,2-Butandiol, 2-Methyl-1,3-propandiol, 1,4-Butandiol, 1,5-Pentandiol, Neopentylglykol, 1,6-Hexandiol, 2-Methylpentan-1,5-diol, 2-Ethylbutan-1,4-diol, 1,10-Decandiol, Diethylenglykol, 2,2,4-Trimethylpentan-1,5-diol, Dipropylenglykol, Tripropylenglykol, 1,4-Dimethylolcyclohexan, 1,6-Dimethylolcyclohexan, 1,1'-Isopropylen-bis-(p-phenyle-phenylenoxy)-di-β-ethanol, 2,2-Bis-(4-hydroxyphenyl)propan (Bisphenol A); Triole, wie Glycerin, Trimethylolethan, Trimethylolpropan, Trimethylolbutan; Tetraole, wie Pentaerythrit, Ditrimethylolpropan; Hexole, wie Erythrit, Sorbit und Dipentaerythrit.

Bevorzugt sind drei- bis sechswertige C₃-C₆-Alkohole, wie Trimethylolpropan, Glycerin, Pentaerythrit und Sorbit.

Geeignet sind auch Oxalkylierungsprodukte. Unter Oxalkylierungsprodukten werden erfindungsgemäß die aus der Umsetzung hydroxylgruppenhaltiger Verbindungen mit Alkylenoxiden oder Alkylenoxidgemischen, wie Ethylenoxid, Propylenoxid, Tetrahydrofuran und/oder Butylenoxid, nach üblichen Verfahren erhältlichen Polymerisationsprodukte verstanden. Bevorzugt sind Oxethylierungs- und Oxpropylierungsprodukte der vorstehend genannten Polyole. Der Oxalkylierungsgrad dieser Polyetherpolyole liegt in der Regel zwischen 1 und 30, bevorzugt zwischen 1 und 10.

Bevorzugt ist oxalkyliertes, insbesondere ethoxyliertes Trimethylolpropan.

Die vorstehend genannten hydroxylgruppenhaltigen Verbindungen mit zwei oder mehreren Hydroxylgruppen pro Molekül weisen (mittlere) Molekulargewichte auf, die im Bereich von vorzugweise 62 bis 4000, insbesondere bei 80 bis 800 und vor allem bei 90 bis 500 liegen.

Als hydroxylgruppenhaltige Verbindungen mit zwei oder mehreren Hydroxygruppen eignen sich auch Polyesterpolyole aus zwei- bis sechswertigen, gegebenenfalls oxalkylierten C₂-C₂₀-Polyolen und zwei- bis vierwertigen C₃-C₃₆-Carbonsäuren oder veresterbaren Derivaten davon. Derartige Polyesterpolyole können in einem separaten Veresterungsschritt hergestellt und dann in der erfindungsgemäßen Stufe a) eingesetzt werden. Sie können aber auch in situ während der in Stufe a) durchgeführten Acrylsäure- oder Methacrylsäureveresterung hergestellt werden, indem man in Stufe a) gleichzeitig zwei- bis sechswertige, gegebenenfalls oxalkylierte C₂-C₂₀-Polyole mit zwei- bis vierwertigen C₃-C₃₆-Carbonsäuren oder veresterbaren Derivaten davon und (Meth)acrylsäure umsetzt. Hierzu eignen sich vornehmlich die zuvor beschriebenen zwei- bis sechswertigen, gegebenfalls oxalkylierten C₂-C₁₀-Polyole und insbesondere deren bevorzugte Vertreter.

Geeignete zwei- bis vierwertige C₃-C₃₆-Carbonsäuren sind insbesondere C₄-C₁₅-Dicarbonsäuren sowie veresterbare Derivate davon, wie Anhydride oder C₁-C₄-Alkylester, beispielsweise Bernsteinsäure, Bernsteinsäureanhydrid, Glutarsäure, Glutarsäureanhydrid, Adipinsäure, Pimelinsäure, Korksäure, Sebacinsäure, Dodecandisäure, Phthalsäure, Phthalsäureanhydrid, Terephthalsäure, Isophthalsäure, Maleinsäure, Maleinsäureanhydrid, Fumarsäure, Citraconsäure, Tetrahydrophthalsäure, Tetrahydrophthalsäureanhydrid, Hexahydrophthalsäure, Hexachlorendomethylentetrahydrophthalsäure, dimere Linolsäure, Trimellithsäure, Trimellithsäureanhydrid, Pyromellithsäure, Pyromellithsäureanhydrid, Citronensäure und Weinsäure, Isomere und Hydrierungsprodukte der vorstehend genannten Carbonsäuren und deren veresterbare Derivate, sowie Gemische davon. Bevorzugt sind Adipinsäure, Phthalsäure, Phthalsäureanhydrid, Maleinsäureanhydrid und Fumarsäure.

Die Polyesterpolyole können in üblicher Weise z.B. durch Veresterung obiger Dicarbonsäuren oder Polycarbonsäuren oder der veresterbaren Derivate davon mit Diolen oder Polyolen hergestellt werden. Als Diole kommen vorzugsweise in Betracht: Ethylenglykol, Propylenglykol-1,2 und -1,3, Butan-diol-1,4, Hexandiol-1,6, Neopentylglykol, Cyclohexandimethanol sowie Polyalkylenglykole insbesondere Polyethylenglykol und Polypropylenglykol. Als Polyole sind in erster Linie Trimethylolpropan, Glycerin oder Pentaerythrit zu nennen.

Als Hydroxylgruppen enthaltende Verbindungen kommen auch Polyether in Frage. Diese werden nach bekannten Verfahren (siehe z. B. Ullmann's Encyclopedia of Industrial Chemistry, Vol. A. 21, 583-584 (1992)) durch Umsetzung von zwei und/oder mehrwertigen Alkoholen mit verschiedenen Mengen an Ethylenoxid und/oder Propylenoxid erhalten. Desgleichen sind auch Polymerisationsprodukte von Tetrahydrofuran oder Butylenoxid verwendbar.

Die zahlenmittleren Molekulargewichte Mn der Polyester bzw. Polyether liegen bevorzugt zwischen 100 und 4000 (Mn bestimmt durch Gelpermeationschromatographie).

Zu den erfindungsgemäß einzusetzenden Hydroxyverbindungen zählen auch Polylactonpolyole aus Lactonen, d.h. cyclischen Hydroxy-C₅-C₁₀-Alkylcarbonsäuren, mit vorzugsweise 6- bis 8-gliedrigen Ringen, insbesondere Caprolactonen und vor allem ε-Caprolacton, und Polyolen, wie den oben genannten, gegebenenfalls oxalkylierten Polyolen, wobei Diole und Triole bevorzugt sind. Derartige Polylactonpolyole können wie die vorstehend genannten Polyesterpolyole nach bekannten Verfahren hergestellt werden.

Bei der Veresterung von (Meth)acrylsäure mit der Hydroxyverbindung werden bevorzugt 0,1 bis 1,5, besonders bevorzugt 0,5 bis 1,4 und ganz besonders bevorzugt 0,7 bis 1,3 Äquivalente, (Meth)acrylsäure, bezogen auf 1 Hydroxy-Äquivalent der Hydroxyverbindungen, eingesetzt. Bei Verwendung eines hydroxylgruppenhaltigen Polyesters ist es z.B. auch möglich, die (Meth)acrylsäure zusammen mit den Ausgangsstoffen des hydroxylgruppenhaltigen Polyesters, z.B. Dicarbonsäuren oder deren Anhydriden, und Diolen bzw. Polyolen vorzulegen und die Ausgangsstoffe zusammen mit der (Meth)acrylsäure in einer Stufe umzusetzen. In diesem Fall beziehen sich die Äquivalente an (Meth)acrylsäure auf die theoretischen nach der Reaktion der Ausgangsstoffe, z.B. der Reaktion von Dicarbonsäuren mit Diolen oder Polyolen, verbleibenden Hydroxy-Äquivalente.

Die Umsetzung der (Meth)acrylsäure mit den Hydroxyverbindungen kann z.B. in Gegenwart eines sauren Veresterungskatalysators, wie z.B. Schwefelsäure, p-Toluolsulfonsäure oder Methansulfonsäure, sowie gegebenenfalls in Gegenwart eines Kohlenwasserstoffs, der mit Wasser ein azeotropes Gemisch bildet, durchgeführt werden. Insbesondere erfolgt die Veresterung bis zu einem Umsatz von mindestens 85%, vorzugsweise 90 bis 95%, der Hydroxylgruppen der Hydroxyverbindung. Die Reaktionstemperatur liegt im Allgemeinen im Bereich von 60 bis 140°C. Geeignete Kohlenwasserstoffe zur azeotropen Entfernung des gebildeten Reaktionswassers sind aliphatische und aromatische Kohlenwasserstoffe, z.B. Alkane und Cycloalkane, wie n-Hexan, n-Heptan, Methylcyclohexan und Cyclohexan, Aromaten, wie Benzol, Toluol und die Xylol-Isomeren, und sog. Spezialbenzine, welche Siedegrenzen zwischen 70 und 140°C aufweisen.

Zur Vermeidung einer vorzeitigen Polymerisation wird die Umsetzung mit (Meth)acrylsäure zweckmäßigerweise in Gegenwart geringer Mengen von Inhibitoren durchgeführt. Dabei handelt es sich um die üblichen, zur Verhinderung einer thermischen Polymerisation verwendeten Verbindungen, z.B. Hydrochinon, Hydrochinonmonoalkylether, 2,6-Di-t-butylphenol, N-Nitrosoamine, Phenothiazine, N-Oxylverbindungen oder Phosphorigsäureester, sowie Gemische aus diesen Inhibitoren. Sie werden im Allgemeinen in Mengen von 0,001 bis 2,0 Gew.-%, vorzugsweise in Mengen von 0,005 bis 0,5 Gew.-%, bezogen auf (Meth)acrylsäure, eingesetzt.

Nach der Veresterung kann das Lösungsmittel, z.B. der Kohlenwasserstoff, aus dem Reaktionsgemisch destillativ, gegebenenfalls unter vermindertem Druck, entfernt werden. Der Veresterungskatalysator kann in geeigneter Weise neutralisiert werden, z.B. durch Zusatz von tertiären Aminen oder Alkalihydroxiden.

Das erhaltene Reaktionsprodukt enthält unumgesetzte (Meth)acrylsäure. Im Allgemeinen liegt die Säurezahl des Reaktionsproduktes im Bereich von 10 bis 200 mg KOH/g.

Zur Entfernung unumgesetzter (Meth)acrylsäure wird in der 2. Stufe das in der 1. Stufe erhaltene Reaktionsprodukt mit einer Epoxidverbindung umgesetzt. Epoxidverbindungen sind solche mit mindestens einer, bevorzugt mindestens zwei, insbesondere zwei oder drei Epoxidgruppen im Molekül.

Als epoxidgruppenhaltige Verbindungen sind sowohl Monoepoxidverbindungen als auch polyfunktionelle Epoxide, insbesondere di-oder trifunktionelle Epoxide, geeignet. Zu nennen sind beispielsweise epoxidierte Olefine, Glycidylester von gesättigten oder ungesättigten Carbonsäuren oder Glycidylether aliphatischer oder aromatischer Polyole. Als Monoepoxidverbindung insbesondere geeignet sind beispielsweise Versaticsäureglycidylester und Alkyl- bzw. Arylglycidylether, wie n-Butylglycidylether, 2-Ethylhexylglycidylether, Phenylglycidylether, o-Kresylglycidylether oder vorzugsweise 1,2-Epoxybutan. Besonders geeignete polyfunktionelle Epoxide sind di- oder trifunktionelle Glycidylether oder Glycidylester. Bevorzugt sind Polyglycidylverbindungen vom Bisphenol-A-Typ oder Glycidylether mehrfunktioneller Alkohole, wie Butan-diol, Glycerin oder Pentaerythrit. Insbesondere bevorzugt sind Bisphenol-A-diglycidylether, beispielsweise Epikote® 828, Butandioldiglycidylether und Pentaerythrittriglycidylether. Beispiele für Handelsprodukte sind Epikote 812® (Epoxidwert: ca. 0,67), Epikote 828 (Epoxidwert: ca. 0,53) und Epikote 162 (Epoxidwert: ca. 0,61) der Firma Shell.

Die Epoxidverbindungen werden dem in der 1. Stufe erhaltenen Reaktionsprodukt im Allgemeinen in Mengen von 1 bis 80 Gew.-%, besonders bevorzugt von 5 bis 20 Gew.-%, bezogen auf das Reaktionsprodukt der 1. Stufe, zugegeben. Ganz besonders bevorzugt werden die Epoxidverbindungen in ungefähr äquimolaren Mengen, bezogen auf die noch vorhandenen Säureäquivalente im Reaktionsprodukt der 1. Stufe, eingesetzt.

Bei der Umsetzung mit Epoxidverbindungen in der 2. Stufe werden überschüssig eingesetzte beziehungsweise nicht umgesetzte (Meth)acrylsäure, daneben aber auch gegebenenfalls noch im Gemisch als Ausgangsstoff vorhandene Dicarbonsäure oder entstandene Halbester von Dicarbonsäuren gebunden.

Die Umsetzung mit Epoxidverbindungen erfolgt bevorzugt bei 90 bis 130°C, besonders bevorzugt bei 100 bis 110°C, und wird vorzugsweise so lange durchgeführt, bis das Reaktionsgemisch eine Säurezahl unter 10, besonders bevorzugt unter 5, mg KOH/g aufweist.

Als Katalysator für die Umsetzung der Epoxidverbindungen mit den Säuregruppen in der 2. Stufe werden erfindungsgemäß stark nucleophile Amine der obigen allgemeinen Formeln als Katalysatoren eingesetzt.

Bevorzugt sind die Dimethylalkylamine wie Dimethylbutylamin, Dimethylethylhexylamin, Dimethyloctylamin, Dimethylundecylamin, Dimethyldodecylamin, Cyclopentyldimethylamin oder Cyclohexyldimethylamin.

Die Aminverbindungen werden bevorzugt in Mengen von 0,01 bis 5 Gew.-%, besonders bevorzugt von 0,1 bis 2 Gew.-%, bezogen auf die Epoxidverbindungen, ganz besonders bevorzugt äquivalent zu dem eingesetzten sauren Veresterungskatalysator, eingesetzt.

Die nach dem erfindungsgemaßen Verfahren erhältlichen strahlungshärtbaren Acrylate eignen sich insbesondere zur Verwendung als oder in strahlungshärtbaren Massen. Sie können verwendet werden als beziehungsweise in Beschichtungsmassen, z.B. Lacken, Druckfarben oder Klebstoffen, als Druckplatten, als Formkörper, zur Herstellung von Photoresisten, in der Stereolithographie oder als Gießmassen z.B. für optische Linsen. Bevorzugt ist die Verwendung zur Beschichtung von flexiblen und gegebenenfalls saugfähigen Substraten, wie Holz und Holzwerkstoffen, beispielsweise Papier und Karton, sowie Leder, oder von nichtflexiblen Substraten aus Metall oder Kunststoff.

Die Anwendung erfolgt im Allgemeinen in Form von Zubereitungen, die neben den strahlungshärtbaren Acrylaten Zusatzstoffe, wie Vernetzer, Verdicker, Verlaufsmittel oder Füllstoffe bzw. Pigmente etc., enthalten.

Die Vernetzung, d.h. die Härtung der Acrylate kann mit energiereicher Strahlung, wie UV-, Elektronen-, Röntgen- oder γ-Strahlung, erfolgen. Dabei ist die UV-Härtung besonders bevorzugt. Sie kann gewünschtenfalls in Gegenwart üblicher Photoinitiatoren, wie z.B. aromatischer Ketoverbindungen, wie Benzophenon, Alkylbenzophenonen, Michler's Keton, Anthron, halogenierten Benzophenonen, Phenylglyoxylsäureestern, Anthrachinon und dessen Derivaten, Benzoinethern, Benzilketalen, Hydroxyalkylphenonen, Acetophenon-Derivaten oder insbesondere Acylphosphinoxiden, wie 2,4,6-Trimethylbenzoyldiphenylphosphinoxid, erfolgen. Dabei können auch Gemische dieser Verbindungen eingesetzt werden. Die Einsatzmenge der Photoinitiatoren beträgt im Allgemeinen etwa 0,01 bis 20 Gew.-%, bevorzugt 0,1 bis 10 Gew.-% und insbesondere 0,5 bis 4 Gew.-%, bezogen auf das Gesamtgewicht der zu härtenden Komponenten. Geeignete Strahlungsquellen sind z.B. Mitteldruckquecksilberdampfstrahler, wie sie etwa auch in "UV Curing: Science and Technology" (Hrsg. S.P. Pappas) beschrieben werden. Erfolgt die Härtung mittels Elektronenstrahlen, so werden im Allgemeinen Beschleunigungsspannungen von 150 bis 500 kV verwendet.

Gegebenenfalls können die erfindungsgemäßen (Meth)acrylsäureester zur Verarbeitung mit weiteren aus der Strahlungshärtung bekannten Reaktivverdünnern versetzt werden. Bei den Reaktivverdünnern handelt es sich im Allgemeinen um copolymerisierbare Monomere, wie (Meth)acrylsäurealkylester oder den entsprechenden Estern der Malein-, Fumar-, Tetrahydrophthal-, Croton-, Isocroton-, Vinylessig- und Itaconsäure. Auch Monomere mit mehr als einer Doppelbindung pro Molekül sind geeignet, wie die Di-, Tri- und Tetra(meth)acrylate von Diolen, Triolen beziehungsweise Tetraolen, sowie Acrylate von alkoxylierten Diolen und Triolen, beispielsweise Ethylenglykol, Diethylenglykol, Propylenglykol, Trimethylenglykol, Neopentylglykol, 1,2-Butandiol, 1,3-Butandiol, 1,6-Hexamethylenglykol, 1,10-Decamethylenglykol, Trimethylolpropan, Pentaerythrit. Beispielhaft seien hier lediglich genannt 4-t-Butylcyclohexylacrylat, Phenoxyethylacrylat, Hexandioldiacrylat, Tripropylenglykoldiacrylat, Trimethylolpropantriacrylat.

Zu weiteren gegebenenfalls geeigneten Additiven gehören Synergisten, wie Amine, Thioverbindungen mit α-endständigen C-H-Gruppen sowie Ether; nichtmitvernetzende Lösungsmittel, wie aromatische Kohlenwasserstoffe oder Ester, beispielsweise Butylacetat; Extender, wie Talkum, Schwerspat oder Silikate; Entschäumer; Verlaufsmittel; filmbildende Hilfsmittel, wie Cellulose-Derivate.

Die erfindungsgemäßen Überzugsmassen können mittels Spritzen, Tauchen, Rakeln, Streichen, Walzen, Fluten oder ähnlicher Maßnahmen auf die zu behandelnden Flächen appliziert werden. Die Filme werden dann durch Strahlung in an sich bekannter Weise gehärtet.

Die Beispiele erläutern die Erfindung ohne sie zu beschränken.

### Beispiele

Für die nachfolgenden Beispiele wurde, wie nachfolgend beschrieben, ein Ester hergestellt und mit Epoxid unter Aminkatalyse umgesetzt.

### Herstellung des Rohesters:

49 Teile eines dreifach ethoxylierten Trimethylolpropans wurden mit 33 Teilen Acrylsäure und 13 Teilen Adipinsäure sowie 25 Teilen Methylcyclohexan vermischt. Weiterhin wurden 0,5 Teile Schwefelsäure, 0,3 Teile Hydrochinonmonomethylether und 0,1 Teile 2,6-Di-tert-butyl-para-kresol zugegeben. Dieses Gemisch wurde unter Rückfluss 6 Stunden erhitzt. 12 Teile Reaktionswasser wurden in dieser Zeit durch ein Trenngefäß entfernt. Nach Ende der Veresterung wurden Methylcyclohexan und überschüssige Acrylsäure abdestilliert bis eine Säurezahl von 42,4 mg KOH/g erreicht war.

Umsetzung der Restsäure mit Epoxiden unter Amin-Katalyse: 54 Teile des Rohesters wurden mit 7 Teilen Epikote 828 (Epoxidharz mit einem Epoxidwert von etwa 0,53) bei 106 bis 108 °C zur Reaktion gebracht. Dabei wurde das Epoxid zugegeben und auf 95 °C aufgeheizt, ehe der Amin-Katalysator zugegeben wurde.

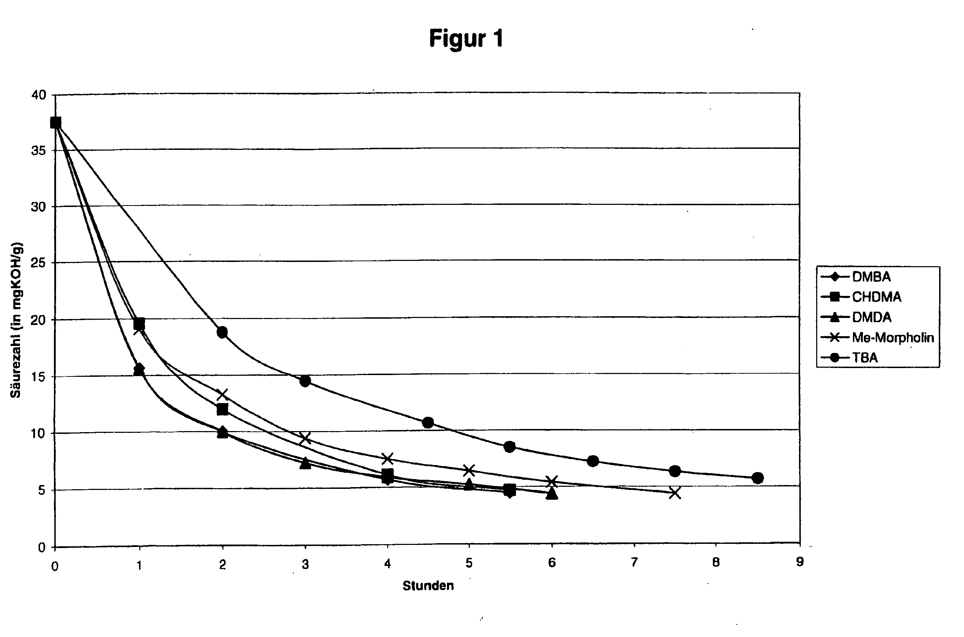

Die Ergebnisse sind in der nachfolgenden Tabelle 1 zusammengestellt und in der Figur 1 graphisch dargestellt.

Es ist ersichtlich, daß bei Verwendung der erfindungsgemäßen Katalysatoren die gewünschte Säurezahl schneller erreicht wird als mit den Vergleichskatalysatoren.

**Tabelle 1**

| | Erfindungsgemäße Beispiele | | | | Vergleichsbeispiel |
|---|---|---|---|---|---|
| Zeit [h] | DMBA Säurezahl [mg KOH/g] | CHDMA Säurezahl [mg KOH/g] | DMDA Säurezahl [mg KOH/g] | Me-Morpholin Säurezahl [mg KOH/g] | TBA Säurezahl [mg KOH/g] |
| 0 | 37,5 | 37,5 | 37,5 | 37,5 | 37,5 |
| 1 | 15,7 | 19,6 | 15,6 | 19,1 | |
| 2 | 10,0 | 12,0 | 10,0 | 13,3 | 18,8 |
| 3 | | | 7,2 | 9,4 | 14,5 |
| 4 | 5,7 | 6,1 | 5,9 | 7,5 | |
| 4,5 | | | | | 10,7 |
| 5 | | | 5,2 | 6,4 | |
| 5,5 | 4,5 | 4,7 | | | 8,5 |
| 6 | | | 4,4 | 5,4 | |
| 6,5 | | | | | 7,2 |
| 7,5 | | | | 4,4 | 6,3 |
| 8,5 | | | | | 5,7 |
| End-SZ | 4,0 | 4,1 | | 4,4 | 4,7 |
| Menge Kat. | 5,7 | 7,2 | 12,1 | 5,7 | 10,5 |

| | | | | | |
|---|---|---|---|---|---|
| Abkürzungen: DMBA Dimethylbutylamin CHDMA Cyclohexyldimethylamin Me-Morpholin N-Methylmorpholin TBA Tributylamin DMDA Dimethyldodecylamin | | | | | |

## Patentansprüche

1. Verfahren zur Herstellung von strahlungshärtbaren (Meth)Acrylaten durch Umsetzung einer Hydroxyverbindung mit Acrylsäure oder Methacrylsäure (erste Stufe), gegebenenfalls in Gegenwart eines sauren Katalysators, und Umsetzung des Reaktionsproduktes mit einer Epoxidverbindung in Gegenwart eines Katalysators (zweite Stufe), **dadurch gekennzeichnet, daß** man als Katalysator in der zweiten Stufe mindestens ein stark nucleophiles Amin der allgemeinen Formeln I oder II 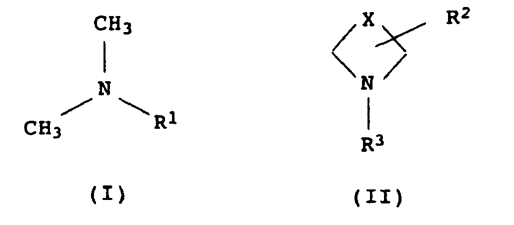 verwendet, wobei
R¹ für eine C₁-C₂₀-Alkylgruppe, bevorzugt C₄-C₁₈-Alkylgruppe, welche gegebenenfalls durch eine oder zwei Phenylgruppen substituiert sein kann, oder eine C₃-C₇-Cycloalkylgruppe steht,
R² für H oder einen C₁-C₁₂-Alkylrest steht,
X für CH₂, (CH₂)₂, (CH₂)₃, (CH₂)₄, CH₂OCH₂ oder CH₂NR⁴CH₂ steht,
R³ für C₁-C₄-Alkyl steht,
R⁴ für H oder C₁-C₄-Alkyl steht.

2. Verfahren nach Anspruch 1, **dadurch gekennzeichnet, dass** man als Katalysator ein Amin der Formel I verwendet.

3. Verfahren nach Anspruch 1 oder 2, wobei R¹ eine C₄-C₁₈-Alkylgruppe ist.

4. Verfahren nach Anspruch 1, **dadurch gekennzeichnet, dass** man als Katalysator Dimethylbutylamin, Cyclohexyldimethylamin oder N-Methylmorpholin verwendet.

5. Verfahren nach einem der Ansprüche 1 bis 4, **dadurch gekennzeichnet, dass** die Aminverbindungen in Mengen von 0,01 bis 5 Gew.-%, bezogen auf die Epoxidverbindungen, eingesetzt werden.

6. Verfahren nach Anspruch 5, **dadurch gekennzeichnet, dass** die Aminverbindungen in Mengen von 0,1 bis 2 Gew.-%, bezogen auf die Epoxidverbindungen, eingesetzt werden.

7. Verfahren nach einem der Ansprüche 1 bis 6, **dadurch gekennzeichnet, dass** man für die Umsetzung der Hydroxyverbindung mit Acrylsäure oder Methacrylsäure einen sauren Katalysator und eine zu dem sauren Katalysator äquivalente Menge des Amins in der 2. Stufe verwendet.

## Claims

1. A process for preparing radiation-curable (meth)acrylates by reacting a hydroxy compound with acrylic acid or methacrylic acid (stage 1), in the presence or absence of an acidic catalyst, and reacting the reaction product with an epoxide compound in the presence of a catalyst (stage 2), which comprises using as second stage catalyst as least one strongly nucleophilic amine of the formula I or II 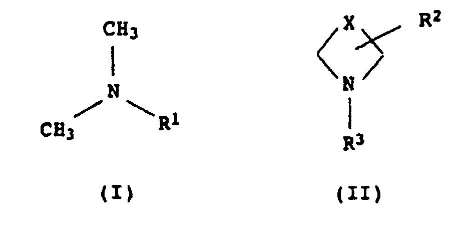 where
R¹ is a C₁-C₂₀ alkyl group, preferably C₄-C₁₈ alkyl group, which may be unsubstituted or substituted by one or two phenyl groups, or is a C₃-C₇ cycloalkyl group,
R² is H or a C₁-C₁₂ alkyl radical,
X is CH₂, (CH₂)₂, (CH₂)₃, (CH₂)₄, CH₂OCH₂ or CH₂NR⁴CH₂,
R³ is C₁-C₄ alkyl,
R⁴ is H or C₁-C₄ alkyl.

2. The process according to claim 1, wherein an amine of the formula I is used as catalyst.

3. The process according to claim 1 or 2, wherein R¹ is a C₄-C₁₈ alkyl group.

4. The process according to claim 1, wherein dimethylbutylamine, cyclohexyldimethylamine or N-methylmorpholine is used as catalyst.

5. The process according to any of claims 1 to 4, wherein the amine compounds are used in amounts of from 0.01 to 5% by weight, based on the epoxide compounds.

6. The process according to claim 5, wherein the amine compounds are used in amounts of from 0.1 to 2% by weight, based on the epoxide compounds.

7. The process according to any of claims 1 to 6, wherein for the reaction of the hydroxy compound with acrylic acid or methacrylic acid an acidic catalyst and an amount of the amine that is equivalent to the acidic catalyst are used in stage 2.

## Revendications

1. Procédé de préparation de (méth)acrylates durcissables par rayonnement, par la réaction d'un composé hydroxy avec l'acide acrylique ou l'acide méthacrylique (première étape), éventuellement en présence d'un catalyseur acide, et la réaction du produit de réaction avec un composé époxyde en présence d'un catalyseur (deuxième étape), **caractérisé en ce que** l'on utilise, en tant que catalyseur dans la deuxième étape, au moins une amine fortement nucléophile de formule générale I ou II 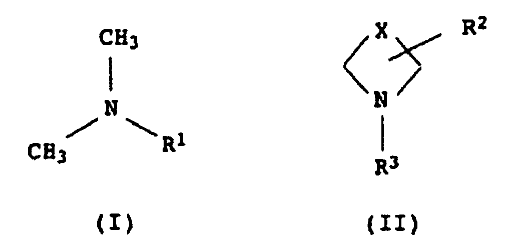 dans lesquelles :
R¹ représente un groupe alkyle en C₁-C₂₀, de préférence, un groupe alkyle en C₄-C₁₈, qui peut être éventuellement substitué par un ou deux groupes phényle, ou un groupe cycloalkyle en C₃-C₇,
R² représente un atome d'hydrogène ou un radical alkyle en C₁-C₁₂,
X représente un groupe CH₂, un groupe (CH₂)₂, un groupe (CH₂)₃, un groupe (CH₂)₄, un groupe CH₂OCH₂ ou un groupe CH₂NR⁴CH₂,
R³ représente un groupe alkyle en C₁-C₄,
R⁴ représente un atome d'hydrogène ou un groupe alkyle en C₁-C₄.

2. Procédé selon la revendication 1, **caractérisé en ce que** l'on utilise une amine de formule I en tant que catalyseur.

3. Procédé selon la revendication 1 ou 2, dans lequel R¹ représente un groupe alkyle en C₄-C₁₈.

4. Procédé selon la revendication 1, **caractérisé en ce que** l'on utilise la diméthylbutylamine, la cyclo-hexyldiméthylamine ou la N-méthylmorpholine en tant que catalyseur.

5. Procédé selon l'une quelconque des revendications 1 à 4, **caractérisé en ce que** les composés amine sont utilisés en quantités de 0,01 à 5 % en poids, par rapport aux composés époxyde.

6. Procédé selon la revendication 5, **caractérisé en ce que** les composés amine sont utilisés en quantités de 0,1 à 2 % en poids, par rapport aux composés époxyde.

7. Procédé selon l'une quelconque des revendications 1 à 6, **caractérisé en ce que** l'on utilise un catalyseur acide et une quantité équivalente au catalyseur acide de l'amine dans la deuxième étape pour la réaction du composé hydroxy avec l'acide acrylique ou l'acide méthacrylique.
